# EUROPEAN PATENT APPLICATION

(11) **EP 4 160 609 A1**
(43) Date of publication of application: **05.04.2023**
(21) Application number: 22199444.5
(22) Date of filing: 03.10.2022
(51) Int. Cl.: G16H 20/40, G16H 40/67

(54) **DETECTION OF DATA ENTRY ERRORS FOR BLOOD SEPARATION SYSTEMS**

(30) Priority: 04.10.2021 US 202163251736 P
(71) Applicant: Fenwal, Inc., Lake Zurich, IL 60047 (US)
(72) Inventor: PLANAS, Samantha M., Lake Zurich, 60047 (US); HIGGINSON, Kathleen M., Lake Zurich, 60047 (US); PATEL, Amit J., Lake Zurich, 60047 (US)
(74) Representative: Maikowski & Ninnemann Patentanwälte Partnerschaft mbB

(57) **Abstract**

A blood separation system is provided with a data entry device, a blood separator, a pump system, and a controller. The controller compares data regarding a blood source that is received by the data entry device and/or calculated values derived from the data to a target range, a maximum value, and/or a minimum value. The controller requests reentry or confirmation of at least a portion of the data when the data or calculated value is outside of the target range, less than the minimum value, and/or greater than the maximum value. The controller instead proceeds with a blood separation procedure when the data or calculated value is not outside of the target range, less than the minimum value, and/or greater than the maximum value or after determining that there have been no data entry errors.

## Description

### Background

### Field of the Disclosure

The present disclosure relates to blood separation systems. More particularly, the present disclosure relates to detection of data entry errors for blood separation systems.

### Description of Related Art

Whole blood is typically separated into its constituents (e.g., red cells, platelets, and plasma) through centrifugation, such as in the AMICUS^{®} separator from Fenwal, Inc. of Lake Zurich, Illinois, which is an affiliate of Fresenius Kabi AG of Bad Homburg, Germany, or other centrifugal separation devices, or a spinning membrane-type separator, such as the AUTOPHERESIS-C^{®} and AURORA^{®} devices from Fenwal, Inc.

Blood separation systems typically include a touch screen or other data entry device (e.g., a computer keyboard) that enables the operator to control the procedure, gather status information, and handle error conditions. A display of the system (e.g., an integrated touch screen or a separate computer screen) will typically display information and prompt the operator to perform different actions. These actions include providing information or data regarding the blood source, such as height and weight (for a living subject) and blood characteristics (e.g., a platelet pre-count) using the user input device. The data entry device may also allow an operator to select one or more procedure parameters, such as a target volume of blood to be drawn or a target volume of a separated blood component (e.g., plasma) to be collected.

One or more of the data entries provided by the operator are typically used by the system in executing a blood separation procedure. For example, the height and weight of a living subject may be used to estimate the total blood volume of the subject, which may be used to assess the volume of blood that may be safely drawn from the subject. Accordingly, proper execution of the procedure relies upon proper input from the operator, such that it would be advantageous to be able to minimize the likelihood of an input error.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood separation system is provided with a data entry device, a blood separator, a pump system, and a controller. The data entry device is configured to receive entered data regarding a blood source, while the blood separator is configured to separate blood into two or more separated blood components. The pump system is configured to convey blood to the blood separator and to convey at least a portion of at least one separated blood component out of the blood separator. The controller is configured to control the blood separator and the pump system to execute a blood separation procedure, with the controller being further configured to compare at least a portion of the entered data received by the data entry device or a calculated value derived from said at least a portion of the entered data received by the data entry device to a target range, a minimum value and/or a maximum value. When said at least a portion of the entered data or said calculated value is outside of said target range, less than said minimum value, and/or greater than said maximum value, the controller requests reentry of said at least a portion of the entered data and/or a different portion of the entered data or requests confirmation of said at least a portion of the entered data and/or a different portion of the entered data. The controller instead proceeds with a blood separation procedure when said at least a portion of the entered data or said calculated value is not outside of said target range, less than said minimum value, and/or greater than said maximum value.

### Brief Description of the Drawings

Fig. 1 is a front perspective view of an exemplary blood separation system according to an aspect of the present disclosure;
Fig. 2 is a rear perspective view of the blood separation system of Fig. 1, with a rear door thereof in an open position;
Fig. 3 is a front perspective view of the blood separation system of Fig. 1, with a fluid flow circuit associated therewith; and
Figs. 4A and 4B illustrate exemplary screens that may be presented on a display or data entry device of the blood separation system of Fig. 1.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

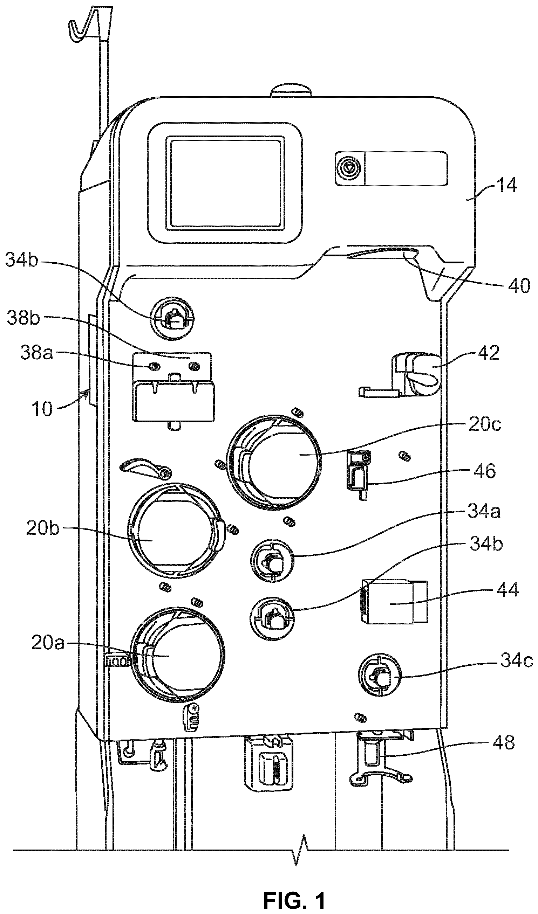

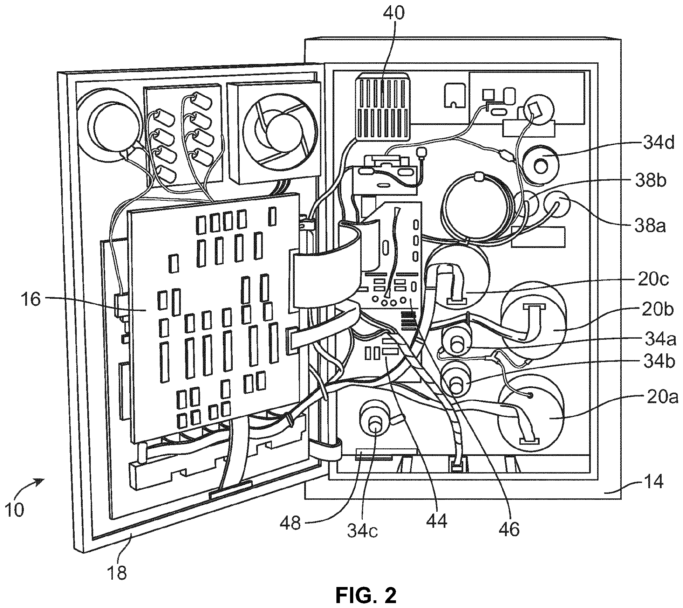

Figs. 1 and 2 illustrate an exemplary automated system 10 for processing blood and blood products. A system of this type (which is similar to the AURORA^{®} devices from Fenwal, Inc.) is described and illustrated in U.S. Patent Application Publication No. 2016/0175509, which is hereby incorporated herein by reference. While such a system is particularly well-suited for performing plasmapheresis, the systems and methods described herein for detecting data entry errors may be used with virtually any automated apheresis or blood processing system, including without limitation those configured to perform red blood cell collection, therapeutic plasma exchange, red blood cell exchange, cell washing, and the like.

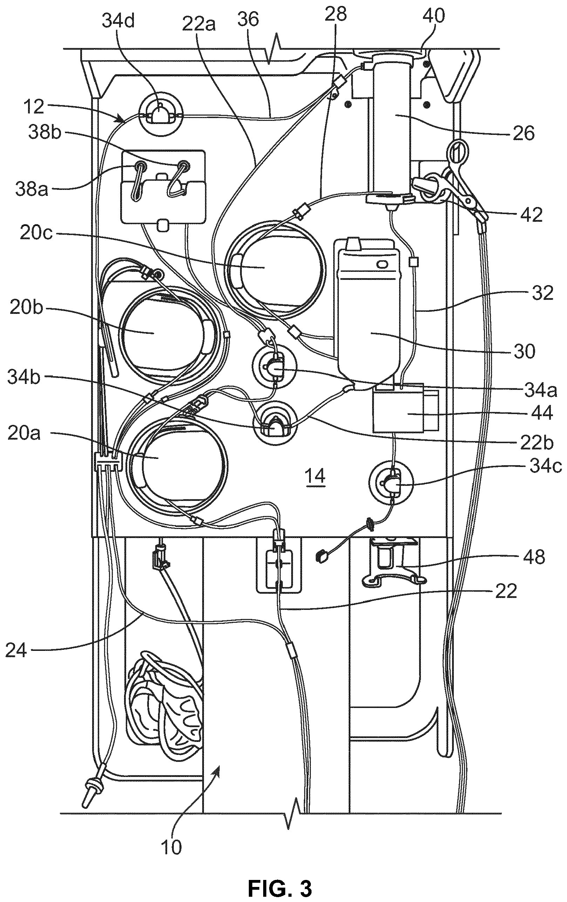

The durable or reusable blood separation system 10 is used in combination with a separate fluid flow circuit (which may be disposable) to separate whole blood (which may or may not be mixed with an anticoagulant) into separated plasma and concentrated cells. Fig. 3 illustrates an exemplary fluid flow circuit 12 mounted onto the blood separation system 10, but it should be understood that the illustrated blood separation system 10 and fluid flow circuit 12 are merely exemplary of such systems and circuits and that differently configured blood processing systems and fluid flow circuits may be provided without departing from the scope of the present disclosure.

The illustrated system 10 includes a cabinet or housing 14, with several components positioned outside of the cabinet 14 (e.g., associated with a front wall or surface or panel of the cabinet 14) and additional components (including a programmable central processing unit or controller 16) and interconnects positioned inside of the cabinet 14, which may be accessed by opening a rear door 18 of the system 10, as shown in Fig. 2. It should be understood that the illustrated system components and the location of the components is merely exemplary, and that additional or different components and different component arrangements may be incorporated into the system without departing from the scope of the present disclosure.

Among the system components positioned on the outside of the cabinet 14, one or more pumps or pump stations 20a-20c may be provided, with the pumps 20a-20c configured to accommodate tubing lines of the fluid flow circuit 12. One of the pumps 20a may be provided as a source/recipient access pump, which may be associated with a source/recipient access line 22 of the fluid flow circuit 12 and operates to draw blood from a blood source and to return fluid to a recipient (which may be the blood source). Another one of the pumps 20b may be provided as an anticoagulant pump, which may be associated with an anticoagulant line 24 of the fluid flow circuit 12 and operates to add anticoagulant from an anticoagulant source or container of the fluid flow circuit 12 to blood drawn from the blood source in the source/recipient access line 22 before the blood enters into a blood separation module or chamber 26 of the fluid flow circuit 12. A third pump 20c may be provided as an outlet pump, which may be associated with a blood cell outlet line 28 and operates to draw separated blood cells from the blood separation chamber 26 and direct it into a collection reservoir 30 after the blood has been separated into blood cells and substantially cell-free plasma in the blood separation chamber 26.

In the illustrated embodiment, the pumps 20a-20c are peristaltic pumps, but it is within the scope of the present disclosure for differently configured pumps, such as diaphragm or other pumps, to be provided. Furthermore, additional or alternative pumps may be provided without departing from the scope of the present disclosure. For example, a pump may be associated with a plasma outlet line 32 of the fluid flow circuit 12 to draw separated plasma from the blood separation chamber 26 after the blood has been separated into cellular blood components and plasma. The illustrated fluid flow circuit 12 employs a single fluid flow tubing or flow path for both drawing blood from a source and flowing or returning it to a recipient, which are carried out intermittently. However, the fluid flow circuit 12 could instead employ separate draw and return flow paths or tubes without departing from the scope of the present disclosure.

In addition to the pumps 20a-20c, the external components of the system 10 may include one or more clamps or valves 34a-34d associated with the tubing lines of the fluid flow circuit 12. The clamps or valves 34a-34d may be variously configured and operate to selectively allow and prevent fluid flow through the associated tubing line. In the illustrated embodiment, one clamp or valve 34a may be provided as a blood source/recipient clamp, which may be associated with a draw branch 22a of the source/recipient access line 22 of the fluid flow circuit 12 to selectively allow or prevent the flow of fluid through the draw branch 22a of the source/recipient access line 22. Another one of the clamps or valves 34b may be provided as a reinfusion clamp or valve, which may be associated with a reinfusion branch 22b of the source/recipient access line 22 downstream of the collection reservoir 30 of the fluid flow circuit 12 to selectively allow or prevent the flow of separated blood cells through the reinfusion branch 22b. A third clamp or valve 34c may be provided as a plasma clamp or valve, which may be associated with the plasma outlet line 32 to selectively allow or prevent the flow of separated plasma through the plasma outlet line 32 and into a separated plasma container. A fourth clamp or valve 34d may be provided as a replacement fluid clamp or valve, which may be associated with a replacement fluid line 36 of the fluid flow circuit 12 to selectively allow or prevent the flow of a replacement fluid out of a replacement fluid source (e.g., a bag or container at least partially filled with saline). Additional or alternative clamps or valves may also be provided without departing from the scope of the present disclosure.

The illustrated system 10 further includes one or more pressure sensors 38a and 38b that may be associated with the fluid flow circuit 12 to monitor the pressure within one or more of the tubing lines of the fluid flow circuit 12 during operation of the pumps 20a-20c and clamps or valves 34a-34d. In one embodiment, one pressure sensor 38a may be associated with a tubing line that draws blood from a blood source and/or directs processed fluid to a fluid recipient, while the other pressure sensor 38b may be associated with a tubing line that directs blood into or separated fluid out of the blood separation chamber 26 to assess the pressure within the blood separation chamber 26, but the pressure sensors 38a and 38b may also be associated with other tubing lines without departing from the scope of the present disclosure. The pressure sensors 38a and 38b may send signals to the system controller 16 that are indicative of the pressure within the tubing line or lines being monitored by the pressure sensor 38a, 38b. If the controller 16 determines that an improper pressure is present within the fluid flow circuit 12 (e.g., a high pressure due to an occlusion of one of the tubing lines), then the controller 16 may instruct one or more of the pumps 20a-20c and/or one or more of the clamps or valves 34a-34d to act so as to alleviate the improper pressure condition (e.g., by reversing the direction of operation of one of the pumps 20a-20c and/or opening or closing one of the clamps or valves 34a-34d). Additional or alternative pressure sensors may also be provided without departing from the scope of the present disclosure.

The system 10 also includes a separation actuator 40 that interacts with a portion of the fluid separation chamber 26 to operate the blood separation chamber 26. A chamber lock 42 may also be provided to hold the blood separation chamber 26 in place with respect to the system cabinet 14 and in engagement with the separation actuator 40. The configuration and operation of the separation actuator 40 depends upon the configuration of the blood separation chamber 26. In the illustrated embodiment, the blood separation chamber 26 is provided as a spinning membrane-type separator, such as a separator of the type described in greater detail in U.S. Patents Nos. 5,194,145 and 5,234,608 or in PCT Patent Application Publication No. WO 2012/125457 A1, all of which are hereby incorporated herein by reference. If provided as a spinning membrane-type separator, the blood separation chamber 26 may include a tubular housing with a microporous membrane positioned therein. An inlet allows a blood to enter into the housing (via the draw branch 22a of the source/recipient access line 22), while a side outlet allows cellular blood components to exit the housing (via the blood cell outlet line 28) and a bottom outlet allows separated plasma to exit the housing (via the plasma outlet line 32) after the blood has been separated into cellular blood components and plasma.

In the illustrated embodiment, the separation actuator 40 is provided as a driver that is magnetically coupled to a rotor on which the membrane is mounted, with the separation actuator 40 causing the rotor and membrane to rotate about the central axis of the housing. The rotating rotor and membrane create Taylor vortices within a gap between the housing and the membrane, which tend to transport the cellular blood components away from the membrane to exit the blood separation chamber 26 via the side outlet, while the separated plasma passes through the membrane toward the central axis of the housing to exit the blood separation chamber 26 via the bottom outlet. It should be understood that the present disclosure is not limited to a particular blood separation chamber and that the illustrated and described fluid separation chamber 26 is merely exemplary. For example, in other embodiments, a centrifugal device that separates blood components based on density, rather than size, may be employed to separate blood into plasma and blood cells.

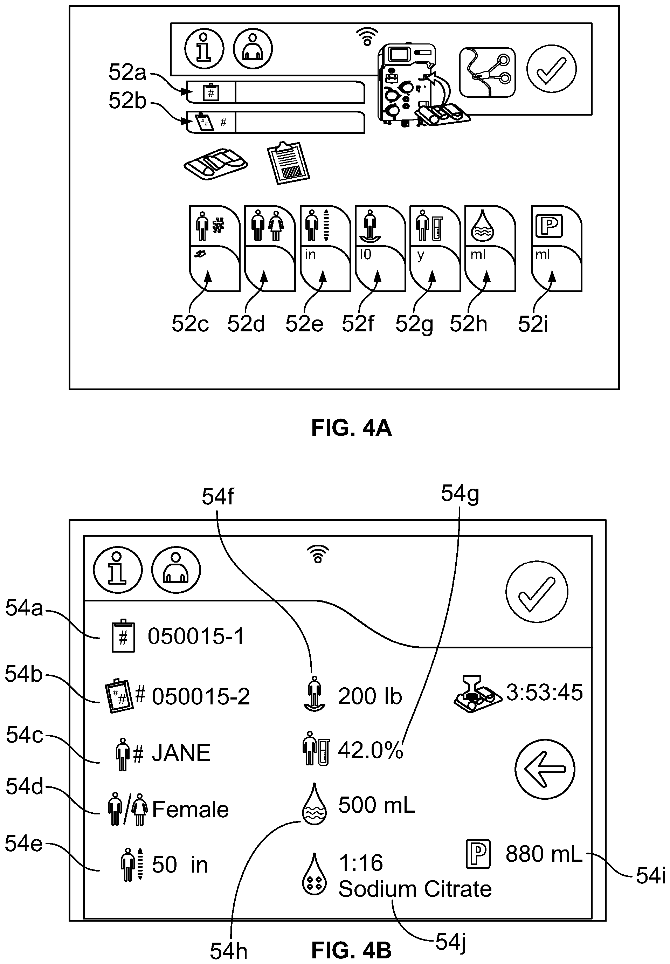

The system 10 may include alternative and/or additional components without departing from the scope of the present disclosure. For example, the illustrated system 10 includes a hemoglobin detector or optical sensor assembly 44 associated with the plasma outlet line 32, a blood cell weigh scale 46 associated with the reservoir 30 and a plasma weight scale 48 associated with the separated plasma container of the fluid flow circuit 12. The illustrated system 10 also includes a data entry device 50 configured as a touch screen for inputting information into the controller 16 and displaying the input information or information originating from the controller 16. While a touch screen is illustrated (with Figs. 4A and 4B showing exemplary images that may be displayed on the touch screen), it should be understood that a differently configured data entry device may also be employed without departing from the scope of the present disclosure.

According to one method of using the blood separation system 10 and fluid flow circuit 12, blood is drawn from a blood source into the blood separation chamber 26 during a draw phase or mode, where it is separated into blood cells and plasma. The separated plasma is retained by the system 10, while the blood cells are returned to a fluid recipient during a return or reinfusion phase or mode. In one embodiment, the draw and return phases are repeatedly alternated (drawing from the blood source, separating the blood into plasma and blood cells, and then returning the blood cells to the fluid recipient) until a target (e.g., a collected volume of separated plasma with or without anticoagulant) is achieved. All of the draw phases and all of the return phases may be identical or may differ from each other. For example, a final draw phase may draw less blood from the blood source than the previous draw phases and a final return phase may infuse a combination of blood cells and replacement fluid to the fluid recipient, whereas the previous return phases return only separated blood cells to the fluid recipient.

As noted above, execution of a blood separation procedure by the controller 16 will typically depend on data entered by an operator using the data entry device 50. Figs. 4A and 4B show exemplary images that may be displayed on a screen of a data entry device 50, with Fig. 4A showing a pre-processing image that requests various information from the operator. The exemplary screen of Fig. 4A includes various fields 52a-52i for receiving and displaying data entered by the operator, which may include: a first field 52a for providing a procedure ID, a second field 52b for providing a donation setup ID, a third field 52c for providing a blood source ID, a fourth field 52d for providing a gender of a human blood source, a fifth field 52e for providing a height of a human blood source, a sixth field 52f for providing a weight of a human blood source, a seventh field 52g for providing information regarding the blood of the blood source (e.g., a hemoglobin or hematocrit level or platelet pre-count), an eighth field 52h for providing a procedure parameter (such as target saline volume), and a ninth field 52i for providing an additional procedure parameter (such as target plasma collection volume or a target combined volume of collected plasma and anticoagulant). It should be understood that the data requested in the screen of Fig. 4A is merely exemplary and that a data entry device 50 may request additional or different information from an operator.

The image of Fig. 4B may be considered as a summary or confirmation screen, which displays the various data entries after they have all been entered by the operator using the screen of Fig. 4A. In addition to nine fields 54a-54i that correspond to the nine fields of 52a-52i (respectively), the screen of Fig. 4B includes an additional field 54j that does not display data entered by the operator, but rather displays a center- or admin-configured value, which is an anticoagulant to whole blood ratio in the illustrated example. Field 54j may instead display a calculated value derived from one or more of the entries provided by the operator using the data entry device 50, or the screen may be provided with one or more additional fields each presenting a different calculated value. This calculated value may be any of a number of possible values including, without limitation, an anticoagulant to whole blood ratio, body mass index of a human source (which may be calculated using the height and weight of the blood source), the total blood volume of the source (which may be estimated using the height and weight of a human blood source according to a known approach, such as use of the Lemmens-Bernstein-Brodsky equation, or a novel approach), the total plasma volume of the source, the extracellular fluid volume of the source, and the estimated time required to complete the procedure. It is within the scope of the present disclosure for a summary or confirmation screen to display a plurality of calculated values and/or for multiple summary or confirmation screens (each displaying one or more calculated values) to be presented.

The controller 16 receives the data entered by the operator using the data entry device 50, along with calculating or being provided with the calculated values. To better ensure that the operator has not made any data entry errors, the controller 16 compares at least a portion of the entered data and/or one or more of the calculated values to a corresponding maximum value, a corresponding minimum value, and/or a target range of corresponding values. For example, an entered weight may be compared to a maximum weight value, a minimum weight value, and/or a range of weight values. In another example, an entered height may be compared to a maximum height value, a minimum weight value, and/or a range of height values. In yet another example, an entered target collection volume (e.g., a volume of collected plasma or a combined volume of collected plasma and anticoagulant) may be compared to a maximum collection volume, a minimum collection volume, and/or a range of collection volumes. In another example, a calculated body mass index may be compared to a maximum body mass index, a minimum body mass index, and/or a range of body mass indices. In yet another example, a calculated total blood volume may be compared to a maximum total blood volume, a minimum total blood volume, and/or a range of total blood volumes. If a data entry or calculated value is less than a corresponding minimum value to which it is compared, more than a corresponding maximum value to which it is compared, and/or outside of the target range of corresponding values to which it is compared (with such data entries and calculated values being referred to herein as "questionable" values), it is an indication that the operator may have made a data entry error.

When there has been a possible data entry error, the controller 16 may prevent initiation of a blood separation procedure, along with responding in any of a variety of ways to attempt to determine whether there has been a data entry error before beginning a blood separation procedure. This may include the controller 16 requesting (e.g., via an image appearing on a display or screen) reentry of each questionable value. For example, if an entered blood source weight, an entered blood source height, and a hematocrit value are all of the questionable values identified by the controller 16, the controller 16 may request that the operator reenter the blood source weight, the blood source height, and the hematocrit value. Alternatively, rather than requesting reentry of each questionable value, the controller 16 may instead request confirmation of each questionable value.

According to another approach, rather than requesting reentry or confirmation of each questionable value, the controller 16 may instead request reentry of only one or more of (but not all of) the questionable values. For example, if an entered blood source weight is greater than a maximum weight value, less than a minimum weight value, and/or outside of a target range of weight values, the result may be a questionable weight value, a questionable body mass index value, and a questionable total blood volume (because body mass index and total blood volume are calculated using the entered weight value). Body mass index and total blood volume are calculated values, rather than entered data, so the controller 16 may request only that the operator reenter the weight of the blood source. Alternatively, rather than requesting reentry of fewer than all of the questionable values, the controller 16 may instead request confirmation of those one or more values (e.g., requesting confirmation that the entered weight value is correct, rather than requesting that the weight value be reentered, in the preceding example).

According to yet another approach, the controller 16 may instead request reentry of at least one value that is not questionable (which will be referred to herein as an "acceptable" value). For example, it is possible for an operator to enter a blood source weight and a blood source height that are each acceptable, but which result in a questionable body mass index. In this case, although the entered weight and entered height are both acceptable, the controller 16 may request reentry of one or both to attempt to determine whether one or both of the values were entered incorrectly. Alternatively, rather than requesting reentry of one or more acceptable values, the controller 16 may instead request confirmation of such values (e.g., requesting confirmation that the entered weight and/or height values are correct, rather than requesting that they be reentered, in the preceding example).

According to another approach, the controller 16 may instead request reentry or confirmation of all of the entered values, regardless of whether a particular entry is acceptable or questionable.

Once the operator has finished reentering or confirming the requested values, the controller 16 recalculates any calculated value that is affected by a reentered value that is different from the corresponding initial value (e.g., recalculating body mass index when a reentered blood source weight is different from an initially entered weight). If at least one reentered value is different from the initially entered value, the controller 16 may be configured to request reentry of some or all of the other entered values before recalculating any calculated values to ensure that the operator has not made any other data entry error that may not have initially been identified by the controller 16.

If there are no longer questionable values after reentry or confirmation of the requested values (i.e., if the operator had initially made a data entry error and then corrected when given a second chance to enter the data or confirmed a questionable value to be correct), the controller 16 may proceed with the selected blood separation procedure. If any questionable values remain, the controller 16 may either again request reentry or confirmation of one or more values (which may be the same values previously reentered or confirmed by the operator or different values) until there are no longer any questionable values or until the controller 16 determines that there have been no data entry errors. The controller 16 may be configured to proceed with the selected procedure after determining that there have been no data entry errors, despite at least one questionable value remaining. The exact approach taken by the controller 16 when determining whether it is acceptable to proceed despite the existence of one or more questionable values (i.e., the manner in which the controller 16 determines that there have been no data entry errors or determines that all remaining questionable values may be ignored and/or treated as acceptable values) may vary without departing from the scope of the present disclosure. For example, in an exemplary embodiment, the controller 16 may be configured to ignore the existence of a questionable value and/or treat a questionable value as an acceptable value and proceed with a procedure when the operator reenters the same values as were initially entered and then confirms the initial/reentered values to be correct.

The particular minimum value, maximum value, and target range of values compared against an entered or calculated value may vary without departing from the scope of the present disclosure. By way of example, an acceptable range of collection volumes may be generated by selecting a target value (e.g., 900 ml) and a particular margin or degree of variation from that target value (e.g., 100 ml above or below the target value or 10% above or below the target value) that is considered to encompass all acceptable values.

The minimum values, maximum values, and/or target ranges of values used by the controller 16 to identify possible data entry errors may be fixed/hard-coded or adjustable/customizable. If one or more of the values is adjustable, the controller 16 may allow an operator (which may include only certain operators or a subset of operators, such as a center manager) to adjust such value(s), which may include the controller 16 limiting the degree to which the operator may adjust a value. Alternatively, rather than (or in addition to) allowing the operator 16 to adjust one or more of the minimum, maximum, and target ranges of values, the controller 16 may instead be configured to automatically adjust such value(s). When one or more values may be adjusted, machine learning principles and/or a data management system may be employed to implement an appropriate adjustment scheme.

As an example of machine learning, the controller 16 may be configured to retain one or more characteristics of a plurality of previous blood sources that have had blood drawn by the system 10. If a particular characteristic is consistently different from an expected value, the controller 16 may employ the retained characteristic when adjusting one or more of the values or determining the degree to which an operator may change one or more of the values. For example, the controller 16 may retain entered data regarding the height of blood sources who have previously have blood separated by the system 10. If the previous blood sources tended to be relatively tall, with correctly entered heights frequently being greater than an initial maximum height value, the controller 16 may determine that it is appropriate to automatically increase the maximum height value, with the particular degree of the adjustment being based on the data retained by the controller 16. In this same exemplary situation, the controller 16 could instead (or additionally) allow for an operator to make larger adjustments to the maximum height value than were initially allowed by the controller 16.

As for a data management system (which may be a central database or server or the like for a center, for example), it may retain one or more characteristics of a larger population of previous blood sources, such as all of the people who have donated blood at a center. If a particular characteristic is consistently different from an expected value, the data management system may transmit data based on the retained characteristic to the controller 16, which the controller 16 may employ when adjusting one or more of the values. For example, the data management system may retain entered data regarding the weight of blood sources previously having blood separated at the center (which may include one or more blood sources who have not previously had their blood separated by the system 10). If the previous blood sources tended to have relatively low weights, with correctly entered weights frequently being less than a minimum weight value, the controller 16 may determine (based on the data from the data management system) that it is appropriate to automatically decrease the minimum weight value, with the particular degree of the adjustment being based on the data received by the controller 16. In this same exemplary situation, the controller 16 could instead (or additionally) use the data received from the data management system to determine that it is acceptable to allow for an operator to make larger adjustments to the minimum weight value than were initially allowed by the controller 16.

The techniques described herein may be used to prevent overcollection or excessive blood draw, as such a situation could be hazardous to a blood source. The techniques may also be used to prevent under-collection which, while not hazardous to a blood source, is undesirable to a blood collection center.

### Aspects

Aspect 1. A blood separation system comprising: a data entry device configured to receive entered data regarding a blood source; a blood separator configured to separate blood into two or more separated blood components; a pump system configured to convey blood to the blood separator and to convey at least a portion of at least one separated blood component out of the blood separator; and a controller configured to control the blood separator and the pump system to execute a blood separation procedure, wherein the controller is further configured to compare at least a portion of the entered data received by the data entry device or a calculated value derived from said at least a portion of the entered data received by the data entry device to a target range, a minimum value, and/or a maximum value, request reentry of said at least a portion of the entered data and/or a different portion of the entered data or request confirmation of said at least a portion of the entered data and/or a different portion of the entered data when said at least a portion of the entered data or said calculated value is outside of said target range, less than said minimum value, and/or greater than said maximum value, and proceed with said blood separation procedure when said at least a portion of the entered data or said calculated value is not outside of said target range, less than said minimum value, and/or greater than said maximum value.

Aspect 2. The blood separation system of Aspect 1, wherein said at least a portion of the entered data includes at least one of an entered height of the blood source and an entered weight of the blood source.

Aspect 3. The blood separation system of Aspect 2, wherein said target range comprises a range of heights, said minimum value comprises a minimum height, and said maximum value comprises a maximum height, and the controller is configured to compare the entered height of the blood source to said range of heights, said minimum height, and/or said maximum height.

Aspect 4. The blood separation system of Aspect 2, wherein said target range comprises a range of weights, said minimum value comprises a minimum weight, and said maximum value comprises a maximum weight, and the controller is configured to compare the entered weight of the blood source to said range of weights, said minimum weight, and/or said maximum weight.

Aspect 5. The blood separation system of Aspect 2, wherein said calculated value comprises a body mass index of the blood source derived from the entered height of the blood source and the entered weight of the blood source, said target range comprises a range of body mass indices, said minimum value comprises a minimum body mass index, and said maximum value comprises a maximum body mass index, and the controller is configured to compare the body mass index of the blood source to said range of body mass indices, said minimum body mass index, and/or said maximum body mass index.

Aspect 6. The blood separation system of Aspect 1, wherein said at least a portion of the entered data includes a hemoglobin level of the blood of the blood source, said target range comprises a range of hemoglobin levels, said minimum value comprises a minimum hemoglobin level, and said maximum value comprises a maximum hemoglobin level, and the controller is configured to compare the entered hemoglobin level to said range of hemoglobin levels, said minimum hemoglobin level, and/or said maximum hemoglobin level.

Aspect 7. The blood separation system of Aspect 1, wherein said at least a portion of the entered data includes a hematocrit level of the blood of the blood source, said target range comprises a range of hematocrit levels, said minimum value comprises a minimum hematocrit level, and said maximum value comprises a maximum hematocrit level, and the controller is configured to compare the entered hematocrit level to said range of hematocrit levels, said minimum hematocrit level, and/or said maximum hematocrit level.

Aspect 8. The blood separation system of Aspect 1, wherein said at least a portion of the entered data includes a platelet pre-count of the blood of the blood source, said target range comprises a range of platelet pre-counts, said minimum value comprises a minimum platelet pre-count, and said maximum value comprises a maximum platelet pre-count, and the controller is configured to compare the entered platelet pre-count to said range of platelet pre-counts, said minimum platelet pre-count, and/or said maximum platelet pre-count.

Aspect 9. The blood separation system of Aspect 1, wherein said calculated value comprises a target collection volume, said target range comprises a range of collection volumes, said minimum value comprises a minimum collection volume, and said maximum value comprises a maximum collection volume, and the controller is configured to compare the target collection volume to said range of collection volumes, said minimum collection volume, and/or said maximum collection volume.

Aspect 10. The blood separation system of Aspect 9, wherein said target collection volume comprises a volume of collected plasma or a combined volume of collected plasma and anticoagulant.

Aspect 11. The blood separation system of Aspect 1, wherein said calculated value comprises a total blood volume of the blood source, said target range comprises a range of total blood volumes, said minimum value comprises a minimum total blood volume, and said maximum value comprises a maximum total blood volume, and the controller is configured to compare the total blood volume of the blood source to said range of total blood volumes, said minimum total blood volume, and/or said maximum total blood volume.

Aspect 12. The blood separation system of Aspect 1, wherein said calculated value comprises a total plasma volume of the blood source, said target range comprises a range of total plasma volumes, said minimum value comprises a minimum total plasma volume, and said maximum value comprises a maximum total plasma volume, and the controller is configured to compare the total plasma volume of the blood source to said range of total plasma volumes, said minimum total plasma volume, and/or said maximum total plasma volume.

Aspect 13. The blood separation system of Aspect 1, wherein said calculated value comprises an extracellular fluid volume of the blood source, said target range comprises a range of extracellular fluid volumes, said minimum value comprises a minimum extracellular fluid volume, and said maximum value comprises a maximum extracellular fluid volume, and the controller is configured to compare the extracellular fluid volume of the blood source to said range of extracellular fluid volumes, said minimum extracellular fluid volume, and/or said maximum extracellular fluid volume.

Aspect 14. The blood separation system of any one of the preceding Aspects, wherein the controller is configured to not allow for adjustment of the target range, the minimum value and/or the maximum value.

Aspect 15. The blood separation system of any one of Aspects 1-13, wherein the controller is configured to allow for adjustment of the target range, the minimum value, and/or the maximum value.

Aspect 16. The blood separation system of Aspect 15, wherein the controller is configured to allow an operator or a subset of operators to adjust the target range, the minimum value, and/or the maximum value.

Aspect 17. The blood separation system of Aspect 16, wherein the controller is configured to limit the adjustments that may be made by the operator or subset of operators to the target range, the minimum value, and/or the maximum value based at least in part on one or more characteristics of previous blood sources retained by the controller.

Aspect 18. The blood separation system of Aspect 16, further comprising a data management system configured to transmit data to the controller, wherein the controller is configured to limit the adjustments that may be made by the operator or subset of operators to the target range, the minimum value, and/or the maximum value based at least in part on the data transmitted to the controller by the data management system.

Aspect 19. The blood separation system of Aspect 18, wherein the data transmitted to the controller from data management system is based at least in part on one or more characteristics of a blood source not previously having blood separated by the blood separation system.

Aspect 20. The blood separation system of Aspect 15, wherein the controller is configured to automatically adjust the target range, the minimum value, and/or the maximum value based at least in part on one or more characteristics retained by the controller of a plurality of blood sources previously having blood separated by the blood separation system.

Aspect 21. The blood separation system of Aspect 15, further comprising a data management system configured to transmit data to the controller, wherein the controller is configured to automatically adjust the target range, the minimum value, and/or the maximum value based at least in part on the data transmitted to the controller by the data management system.

Aspect 22. The blood separation system of Aspect 21, wherein the data transmitted to the controller from the data management system is based at least in part on one or more characteristics of a blood source not previously having blood separated by the blood separation system.

Aspect 23. The blood separation system of any one of the preceding Aspects, wherein the controller is configured to request reentry of all of the entered data when said at least a portion of the entered data or said calculated value is outside of said target range, less than said minimum value, and/or greater than said maximum value.

Aspect 24. The blood separation system of any one of the preceding Aspects, wherein the controller is configured to request reentry of said at least a portion of the entered data when said at least a portion of the entered data or said calculated value is outside of said target range, less than said minimum value, and/or greater than said maximum value, and when the reentered data is different from said at least a portion of the entered value, request reentry of said different portion of the entered data.

Aspect 25. The blood separation system of any one of the preceding Aspects, wherein the controller is configured to determine whether an entered datum and/or calculated value outside of said target range, less than said minimum value, and/or greater than said maximum value is acceptable, and proceed with said blood separation procedure when all entered data and/or calculated values outside of said target range, less than said minimum value, and/or greater than said maximum value are determined to be acceptable, wherein the determination of whether an entered datum and/or calculated value outside of said target range, less than said minimum value, and/or greater than said maximum value is acceptable is made after at least one entered datum and/or calculated value has been reentered and/or confirmed at least once.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood separation system comprising:
a data entry device configured to receive entered data regarding a blood source;
a blood separator configured to separate blood into two or more separated blood components;
a pump system configured to convey blood to the blood separator and to convey at least a portion of at least one separated blood component out of the blood separator; and
a controller configured to control the blood separator and the pump system to execute a blood separation procedure, wherein the controller is further configured to
compare at least a portion of the entered data received by the data entry device or a calculated value derived from said at least a portion of the entered data received by the data entry device to a target range, a minimum value, and/or a maximum value,
request reentry of said at least a portion of the entered data and/or a different portion of the entered data or request confirmation of said at least a portion of the entered data and/or a different portion of the entered data when said at least a portion of the entered data or said calculated value is outside of said target range, less than said minimum value, and/or greater than said maximum value, and
proceed with said blood separation procedure when said at least a portion of the entered data or said calculated value is not outside of said target range, less than said minimum value, and/or greater than said maximum value.

2. The blood separation system of claim 1, wherein said at least a portion of the entered data includes at least one of an entered height of the blood source and an entered weight of the blood source.

3. The blood separation system of claim 2, wherein
said calculated value comprises a body mass index of the blood source derived from the entered height of the blood source and the entered weight of the blood source,
said target range comprises a range of body mass indices, said minimum value comprises a minimum body mass index, and said maximum value comprises a maximum body mass index, and
the controller is configured to compare the body mass index of the blood source to said range of body mass indices, said minimum body mass index, and/or said maximum body mass index.

4. The blood separation system of claim 1, wherein said at least a portion of the entered data includes at least one of a hemoglobin level of the blood of the blood source, a hematocrit level of the blood of the blood source, a platelet pre-count of the blood of the blood source, a total blood volume of the blood source, a total plasma volume of the blood source, and an extracellular fluid volume of the blood source.

5. The blood separation system of claim 1, wherein
said calculated value comprises a target collection volume,
said target range comprises a range of collection volumes, said minimum value comprises a minimum collection volume, and said maximum value comprises a maximum collection volume, and
the controller is configured to compare the target collection volume to said range of collection volumes, said minimum collection volume, and/or said maximum collection volume.

6. The blood separation system of claim 5, wherein said target collection volume comprises a volume of collected plasma or a combined volume of collected plasma and anticoagulant.

7. The blood separation system of any one of the preceding claims, wherein the controller is configured to allow an operator or a subset of operators to adjust the target range, the minimum value, and/or the maximum value.

8. The blood separation system of claim 7, wherein the controller is configured to limit the adjustments that may be made by the operator or subset of operators to the target range, the minimum value, and/or the maximum value based at least in part on one or more characteristics of previous blood sources retained by the controller.

9. The blood separation system of claim 7, further comprising a data management system configured to transmit data to the controller, wherein the controller is configured to limit the adjustments that may be made by the operator or subset of operators to the target range, the minimum value, and/or the maximum value based at least in part on the data transmitted to the controller by the data management system.

10. The blood separation system of any one of claims 1-6, further comprising a data management system configured to transmit data to the controller, wherein the controller is configured to automatically adjust the target range, the minimum value, and/or the maximum value based at least in part on the data transmitted to the controller by the data management system.

11. The blood separation system of any one of claims 9-10, wherein the data transmitted to the controller from the data management system is based at least in part on one or more characteristics of a blood source not previously having blood separated by the blood separation system.

12. The blood separation system of any one of claims 1-6, wherein the controller is configured to automatically adjust the target range, the minimum value, and/or the maximum value based at least in part on one or more characteristics retained by the controller of a plurality of blood sources previously having blood separated by the blood separation system.

13. The blood separation system of any one of the preceding claims, wherein the controller is configured to request reentry of all of the entered data when said at least a portion of the entered data or said calculated value is outside of said target range, less than said minimum value, and/or greater than said maximum value.

14. The blood separation system of any one of the preceding claims, wherein the controller is configured to
request reentry of said at least a portion of the entered data when said at least a portion of the entered data or said calculated value is outside of said target range, less than said minimum value, and/or greater than said maximum value, and
when the reentered data is different from said at least a portion of the entered value, request reentry of said different portion of the entered data.

15. The blood separation system of any one of the preceding claims, wherein the controller is configured to
determine whether an entered datum and/or calculated value outside of said target range, less than said minimum value, and/or greater than said maximum value is acceptable, and
proceed with said blood separation procedure when all entered data and/or calculated values outside of said target range, less than said minimum value, and/or greater than said maximum value are determined to be acceptable, wherein the determination of whether an entered datum and/or calculated value outside of said target range, less than said minimum value, and/or greater than said maximum value is acceptable is made after at least one entered datum and/or calculated value has been reentered and/or confirmed at least once.
